# EUROPÄISCHE PATENTSCHRIFT

(11) **EP 2 522 310 B1**
(45) Veröffentlichungstag und Bekanntmachung des Hinweises auf die Patenterteilung: **13.04.2016**
(21) Anmeldenummer: 12003586.0
(22) Anmeldetag: 08.05.2012
(51) Int. Cl.: A61F 2/30, A61F 2/36

(54) **Form zur Herstellung von Spacern für Endoprothesen**
Mould for producing spacers for endoprosthetics
Moule de fabrication d'espaceurs pour endoprothèses

(30) Priorität: 10.05.2011 DE 102011101084
(43) Veröffentlichungstag der Anmeldung: 14.11.2012
(73) Patentinhaber: Heraeus Medical GmbH, 61273 Wehrheim (DE)
(72) Erfinder: Wüst, Edgar, 64823 Gross-Umstadt (DE)
(74) Vertreter: Panten, Kirsten

(56) Entgegenhaltungen:
- DE-U1-202009 012 964
- US-A1- 2010 102 484

## Beschreibung

Die vorliegende Erfindung betrifft eine Form zur Herstellung von Spacern für Endoprothesen, sie betrifft insbesondere eine Form zur Herstellung von Spacern für Hüftendoprothesen.

In Deutschland werden zurzeit ca. 200.000 Hüfttotalendoprothesen pro Jahr implantiert. Als relevante Komplikationen können Früh- und Spätinfektionen des Implantatlagers auftreten. Die Infektrate wird dabei auf 2-6% beziffert, je nachdem ob es sich um eine Primärimplantation oder eine Revisionsoperation handelt. Die vermutete Dunkelziffer liegt weitaus höher, insbesondere bzgl. kritischer Keime (z. B. MRSA). Kommt es zur Infektion im Bereich des künstlichen Gelenkes, wird im ersten Schritt versucht, den Infekt mittels konsequenter chirurgischer Sanierung kombiniert mit testgerechten Antibiotika zu behandeln. In sehr vielen Fällen ist dies alleine jedoch nicht wirksam. Die infizierte Prothese muss dann operativ entfernt werden. Dabei wird gleichzeitig der durch die Infektion kompromittierte Knochen mit entfernt. Je weiter fortgeschritten die Infektion, desto größer die Knochendefekte.

Nachdem die infizierte Prothese entfernt wurde, platziert man, um die Stabilität des Gelenkes aufrecht zu erhalten, an Stelle der Prothese einen sogenannten Platzhalter oder Spacer. Während der Behandlung der Infektion wird das Gelenk des Patienten bis zum negativen Keimnachweis alle 3-5 Tage revidiert, debridiert und gespült, Abstriche entnommen und der Spacer ggf. ausgetauscht, parallel zur testgerechten antibiotischen Behandlung. Die Dauer der Behandlung hängt von verschiedenen Faktoren ab, wie zum Beispiel dem Ansprechen der Bakterien auf das Antibiotikum, der Verfassung des Patienten, dem Schweregrad der Infektion etc.. Nach belegter Sanierung der Infektion wird dem Patienten eine Revisionsprothese implantiert. Die zurzeit auf dem Markt befindlichen Spacer bestehen aus Kunststoff (PMMA). Im Inneren kann sich eine Verstärkung aus Edelstahl befinden (siehe z. B. bei Hüftgelenk-Spacern). Die Geometrie der Hüftgelenk-Spacer orientiert sich im Groben am Design einer Primärprothese. Unberücksichtigt bleiben dabei die individuellen Zuschnitte am Hüftgelenk, die von der ursprünglich implantierten Prothese vorgegeben sind.

Alternativ zu den oben genannten Spacern verwenden manche Operateure Platzhalter, die während der Operation eigenhändig aus Knochenzement hergestellt werden. Dem Knochenzement kann während der Herstellung wahlweise antibiotisch wirksame Bestandteile zugemischt werden. Zur Herstellung dieser Spacer werden Formen benötigt.

Eine solche Form für Spacer-Implantate wird in US 2007/0222114 A1 beschrieben. Diese Form besteht aus einer Vielzahl von Formsegmenten, die miteinander verbunden werden. Durch die Vielzahl der Segmente kann die Form ziemlich genau an die anatomischen Besonderheiten des Patienten angepasst werden. Die Formsegmente werden mittels Schraubschellen aneinandergefügt. Durch Kanäle in der Form wird der Knochenzement eingeführt. Durch diesen komplizierten Aufbau ist es sehr aufwändig, die Formsegmente zusammenzufügen und später den ausgehärteten Knochenzement wieder zu entnehmen.

In WO 2009/073781 A2 wird eine Form für einen Hüftspacer vorgeschlagen, die aus zwei Teilen besteht, die in Bezug zueinander verschoben werden können, um die Länge des Hüftschaftes anpassen zu können. Der Teil, der den Hüftschaft bildet besteht aus zwei spiegelbildlichen Halbschalen, die auseinandergenommen werden können. Die Knochenzementmasse wird über eine Zuführöffnung in den Hohlraum eingeführt. Die einzelnen Formteile sind mit Hilfe von Schraubverbindungen miteinander verbunden. Zur Entnahme des Spacers müssen sämtliche Schrauben gelöst werden. Damit erfordert die intraoperative Herstellung relativ viel Zeit mit dem Risiko einer erhöhten Infektionsrate.

Dokument US 2010/0102484 A1 zeigt die Merkmale des Oberbegriffs des unabhängigen Anspruchs 1.

Aufgabe der Erfindung ist es daher, eine Form für Spacer bereitzustellen, die einfach und schnell mit Knochenzement gefüllt werden kann, und aus der der fertige Spacer genauso einfach und schnell wieder entnommen werden kann.

Diese Aufgabe wird durch den Gegenstand des unabhängigen Anspruchs gelöst. Bevorzugte Ausführungsformen sind in den abhängigen Ansprüchen wiedergegeben.

Die Aufgabe wird durch eine Form für einen Endoprothesenspacer gemäß Anspruch gelöst. Weiterbildungen der Erfindung sind durch die Unteransprüche definiert.

Die erfindungsgemäße Form für einen Spacer besteht aus mindestens zwei Teilformen, bevorzugt aus zwei Teilformen, welche zusammengesteckt werden können. Das bedeutet, dass die Teilformen an dem Ende, an dem sie zusammengesteckt werden, so ausgebildet sind, dass sie passgenau ineinandergeführt werden können. Bei einer besonders bevorzugten Ausführungsform der Erfindung handelt es sich um einen Hüftgelenkspacer, der am Oberschenkelhals zusammengesetzt wird.

Erfindungsgemäß weist eine der Teilformen, bevorzugt die Teilform für den Hüftgelenkkopf einen Aufnahmeabschnitt auf, in den ein Einschubabschnitt der anderen Teilform, bevorzugt der Teilform für den Hüftgelenkschaft, einführbar ist. Zumindest einer von beiden Abschnitten, entweder der Aufnahmeabschnitt oder der Einschubabschnitt, weist Verbindungselemente auf, die integriert mit der Teilform ausgebildet sind und eine form-, kraft- oder form- und kraftschlüssige Verbindung mit der zweiten Teilform bilden können.

In einer bevorzugten Ausführungsform weist eine Teilform mehrere Verbindungselemente auf, die in Abständen derart ausgebildet sind, dass der Aufnahmeabschnitt, der über den Einschubabschnitt geschoben wird, in unterschiedlichen Positionen fixiert werden und so die Länge des Oberschenkelhalses angepasst werden. Bevorzugt sind 2 bis 10 Verbindungelemente, besonders bevorzugt 2 bis 5 Verbindungselemente. Besonders bevorzugt sind an der Teilform mit dem Einschubabschnitt eine Mehrzahl Verbindungselemente ausgebildet.

Bevorzugt handelt es sich bei den Verbindungselementen um Rastelemente am Aufnahmeabschnitt, die mit komplementären Verbindungselementen der zweiten Teilform in der der Einschubrichtung entgegengesetzten Richtung eine formschlüssige Verbindung bilden.

Besonderes bevorzugt sind die Rastelemente an Laschen ausgebildet, die an dem Aufnahmenabschnitt angebracht sind. Bevorzugt handelt es sich bei den Rastelementen um Vorsprünge, wie zum Beispiel Haken. Bei den Verbindungselementen der zweiten Teilform kann es sich zum Beispiel um Rillen handeln, in die die Haken passen. Durch ihre längliche Ausbildung besitzen die Laschen eine gewisse Elastizität, die es ermöglicht, dass sie über die zweite Teilform geschoben werden können. Diese Elastizität kann jedoch auch durch die Verwendung eines elastischen Materials gewährleistet sein.

Bevorzugt befinden sich die Rastelemente an Laschen, die über den Aufnahmeabschnitt hinausragen. Die Laschen sollten elastisch sein, so dass das Einführen des Einschubabschnitts erleichtert wird.

Denkbar ist jedoch auch, dass diese Vorsprünge nicht an Laschen, sondern direkt im Aufnahmeabschnitt der ersten Teilform ausgebildet sind. Die Vorsprünge können auch durch einen umlaufenden vorspringenden Rand gebildet sein, der in eine umlaufende Nut in der anderen Teilform greift.

Damit die Laschen mit den Rastelementen sicher auf die komplementären Verbindungselemente ausgerichtet werden können, können die Querschnitte des rohrförmigen Aufnahmeabschnitts und des rohrförmigen Einschubabschnitts mehreckig, wie zum Beispiel viereckig oder sechseckig, oder auch oval sein. Damit kann die erste Teilform auch nicht nur geringfügig im Hinblick auf die zweite Teilform verdreht werden. Damit der Querschnitt annähernd den anatomischen Vorgaben entspricht, sollten die Kanten abgerundet sein.

Anstelle der Rastelemente können auch Federelemente an dem Aufnahmeabschnitt oder dem Einschubabschnitt ausgebildet sein, die die zweite Teilform durch Federkraft und damit kraftschlüssig in Position halten. In diesem Fall muss die zweite Teilform keine komplementären Elemente aufweisen. Von Vorteil wäre eine aufgeraute Oberfläche.

Bei der Herstellung des Spacers werden zunächst die beiden Formteile gefüllt und anschließend zusammengesteckt und über die Rastelemente fixiert.

Jede Teilform besteht bevorzugt aus mehreren Elementen, die zusammengesetzt werden können. Insbesondere besteht jede Teilform aus zwei Halbschalelementen, die einen flachen Rand aufweisen. Die Halbschalelemente können mit ihren Rändern aufeinandergelegt werden. Bevorzugt sind an den Rändern lösbare Befestigungsmittel angeordnet, die die Halbschalelemente aneinander befestigen. Bei diesen Befestigungsmitteln handelt es sich bevorzugt um Haken an einem Halbschalelement, die in Öffnungen des zweiten Halbschalelementes greifen. Die Haken sind bevorzugt elastisch und abgerundet, so dass beim Aufwenden einer bestimmten Kraft die Halbschalelemente auseinandergezogen werden können, um den Spacer entnehmen zu können.

Die Formen können aus jedem beliebigen biokompatiblen Material hergestellt werden. Es gibt verschiedene Polymere, die für diese Anwendungen geeignet sind. Besonders geeignet sind formstabile, harte Materialien. Beispiele hierfür sind Polyethylen, Polypropylen und Polyetherketon. Diese Materialien haben eine ausreichende Festigkeit, so dass sie sich während der Herstellung der Spacer nicht verformen. Prinzipiell sind alle biokompatiblen Materialien geeignet, aus denen sich die Formen herstellen lassen, unter der Voraussetzung, dass sie keine chemische Reaktion mit Knochenzement oder Antibiotikum eingehen. Da die Form aus zwei Teilformen besteht, die separat gefüllt werden, kann sie auch intransparent sein.

Wahlweise kann vor dem Füllen der Form auch ein Verstärkungselement eingesetzt werden. Es handelt sich dabei bevorzugt um ein längliches flaches Element, das in seiner äußeren Form dem Querschnitt der Form folgt, wobei jedoch ausreichend Abstand zur Form bleibt. Das Verstärkungselement ist bevorzugt aus Edelstahl gefertigt. Es weist bevorzugt Löcher auf, so dass der Knochenzement beim Füllen der Form in diese Löcher eindringen kann. Nach dem Aushärten ist das Verstärkungselement fest mit dem Knochenzement verbunden.

Im Weiteren wird die vorliegende Erfindung anhand der beiliegenden Zeichnungen genauer beschrieben. Es zeigen:
Fig. 1 eine perspektivische Darstellung eines Ausführungsbeispiels einer erfindungsgemäßen Form für einen Hüftspacer,
Fig. 2 eine perspektivische Darstellung eines Ausführungsbeispiels einer erfindungsgemäßen halben Form für einen Hüftspacer, und
Fig. 3 eine seitliche Ansicht eines Ausschnitts aus einer erfindungsgemäßen Form.

In den Figuren bezeichnen dieselben Bezugszeichen gleiche oder funktionsgleiche Komponenten, soweit nichts Gegenteiliges angegeben ist.

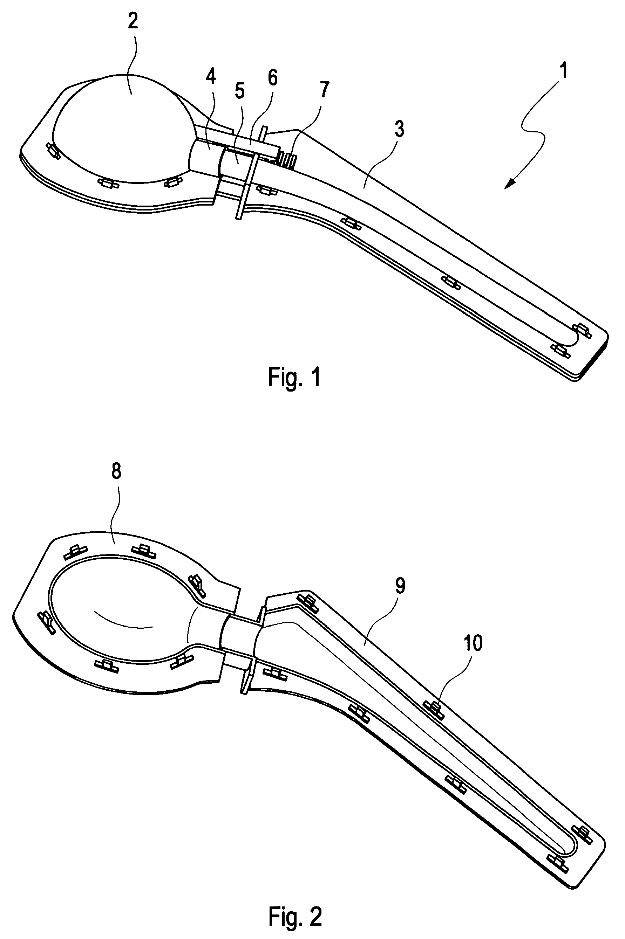

Fig. 1 zeigt die perspektivische Ansicht einer Form 1 zur Herstellung eines Hüftgelenkspacers. Die Form besteht aus zwei Teilformen, die ineinander gesteckt werden können. Die erste Teilform 2 dient zur Ausbildung des Oberschenkelkopfes, während die zweite Teilform 3 zur Ausbildung des Oberschenkelschaftes dient.

Um den Spacer genau an die anatomischen Bedingungen des Patienten anpassen zu können, stehen unterschiedliche Teilformen zur Verfügung. Die verschiedenen Teilformen für den Oberschenkelkopf können zum Beispiel unterschiedliche Durchmesser haben, aber es sind auch unterschiedliche Formen denkbar. Die Teilformen für den Oberschenkelschaft können sich z.B. in Durchmesser und Länge unterscheiden. Es besteht somit eine Vielzahl von Möglichkeiten, wie Teilformen für den Oberschenkelkopf mit Teilformen des Oberschenkelschaftes kombiniert werden. Damit steht eine große Anzahl unterschiedlicher Spacer für Hüftendoprothesen zur Verfügung.

Beide Teilformen werden an einem Abschnitt zusammengefügt, der der Ausbildung des Oberschenkelhalses dient. Die erste Teilform 2 für den Oberschenkelkopf weist einen Aufnahmeabschnitt 4 auf, in den ein Einschubabschnitt 5 der zweiten Teilform 3 eingeführt werden kann. Nach dem Zusammenfügen der Teilformen 2 und 3 bildet der Aufnahmenabschnitt 4 zusammen mit einem Eischubabschnitt 5 an der Teilform 3 für den Oberschenkelschaft den Oberschenkelhals aus. Aufnahmeabschnitt 4 und Einschubabschnitt 5 können unterschiedlich weit übereinander geschoben werden und in verschiedenen Positionen fixiert werden. So kann auch die Länge des Oberschenkelhalses nach Bedarf eingestellt werden.

Die beiden Teilformen werden mit Hilfe von Rastelementen 6,7 aneinander fixiert. In der vorliegenden Ausführungsform sind am Aufnahmeabschnitt 4 der Teilform 2 für den Oberschenkelkopf Laschen 6 befestigt, die über den Aufnahmeabschnitt 4 hinausragen.

In Figur 2 ist eine halbe Form für die Herstellung eines Spacers dargestellt. Die Darstellung zeigt den Zustand nach Entnahme des Spacers. Die Elemente der jeweiligen Teilformen 2,3 können ebenfalls auseinandergenommen werden, so dass zur Entnahme des Spacers die Form 1 der Länge nach geöffnet wird. Es ist zu sehen, dass ein Halbschalelement 8 der Teilform 2 für den Oberschenkelkopf und ein Halbschalelement 9 der Teilform 3 für den Oberschenkelhals mehrere Haken 10 aufweisen. Wie in Figur 1 zu sehen ist, greifen diese Haken 10 in geschlossenem Zustand in Öffnungen der beiden anderen Halbschalelemente, die in Fig. 2 nicht dargestellt sind.

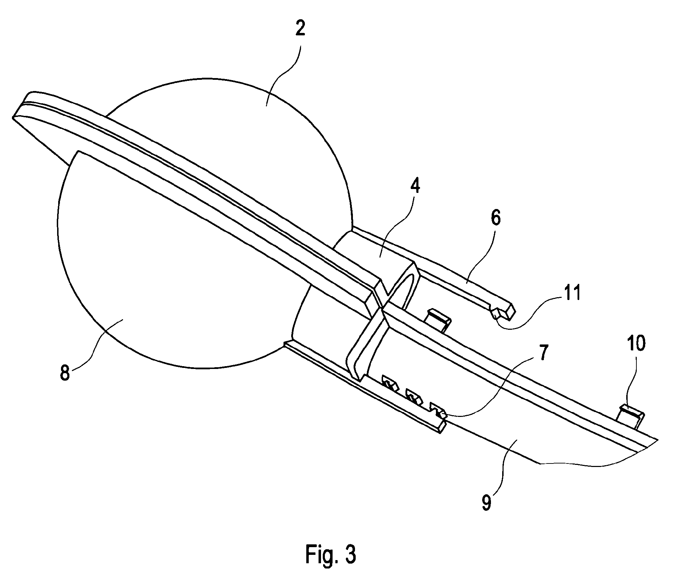

Figur 3 zeigt die Verbindungselemente 6,7, die dazu dienen, die erste Teilform 2 und die zweite Teilform 3 aneinander zu befestigen, im Detail. Die Laschen 6 weisen Vorsprünge 11 auf, welche in Rillen 7 greifen, die an der Teilform 3 für den Oberschenkelschaft angebracht sind. Bevorzugt sind an der Teilform 3 für den Oberschenkelschaft mehrere Rillen angeordnet, so dass der Aufnahmeabschnitt 4 unterschiedlich weit über den Einschubabschnitt 5 geschoben werden kann und dort in verschiedenen Positionen fixiert werden kann.

Die erfindungsgemäßen Formen haben den Vorteil, dass die Verbindungselemente integriert mit den Teilformen ausgebildet sind, so dass die Handhabung erheblich vereinfacht ist. Die beiden Teilformen müssen lediglich einfach zusammengeschoben werden. Die Verbindungselemente greifen gleichzeitig und automatisch, so dass das Zusammensetzen der Form einfach und schnell zu bewerkstelligen ist. Genauso einfach und schnell kann der Spacer entnommen werden, indem die Elemente der Teilformen auseinandergezogen werden. Bei der der Ausführungsform entsprechenden Form für einen Hüftgelenkspacer besteht die Form lediglich aus vier größeren Elementen.

### Bezugszeichenliste

- 1: Form für einen Hüftgelenkspacer
- 2: Teilform für Oberschenkelkopf
- 3: Teilform für Oberschenkelschaft
- 4: Aufnahmeabschnitt
- 5: Einschubabschnitt
- 6: Lasche
- 7: Rastelemente
- 8: Halbschale
- 9: Halbschale
- 10: Haken
- 11: Verbindungselemente

## Patentansprüche

1. Form zur Herstellung von Spacern für Endoprothesen enthaltend mindestens zwei Teilformen,
wobei
die erste Teilform (2) an einer offenen Seite einen Aufnahmeabschnitt (4) aufweist, in den ein Einschubabschnitt (5) an der offenen Seite der zweiten Teilform (3) einschiebbar ist,
wobei die erste Teilform (2) Verbindungselemente (11) aufweist, die integriert in die erste Teilform (2) ausgebildet sind,
wobei die Form zur Herstellung eines Spacers für eine Hüftendo rothese dient, wobei eine der beiden Teilformen (2,3) die Form eines Oberschenkulkopfes mit Oberschenkelhalsansatz umgibt und die anderen Teilform (3,2) die Form eines Schaftes mit Oberschenkelhalsansatz umgibt,
wobei an der zweiten Teilform (3) eine Mehrzahl von Verbindungselementen ausgebildet ist, und wobei der Aufnahmeabschnitt (4) über den Einschubabschnitt (5) geschoben wird,
**dadurch gekennzeichnet, dass**
die Verbindungselemente (11) der ersten Teilform (2) am Außenumfang der zweiten Teilform anareifen und die zweite Teilform (3) über eine form- und/oder kraftschlüssige Verbindung in Position halten können, derart dass der Aufnahmeabschnitt (4), in unterschiedlichen Positionen fixierbar und so an die Länge des Oberschenkelhalses anpassbar ist oder derart, dass an dem Aufnahmeabschnitt (4) der ersten Teilform Federelemente ausgebildet sind und die zweite Teilform keine komplementären Elemente aufweist.

2. Form nach Anspruch 1,
**dadurch gekennzeichnet, dass**
die Verbindungselemente (11) Rastelemente sind, die in komplementäre Elemente (7) an der zweiten Teilform (3) passen.

3. Form nach Anspruch 2,
**dadurch gekennzeichnet, dass**
die zweite Teilform (3) zu jedem Rastelement (11) der ersten Teilform (2) mehrere nebeneinender angeordnete komplementäre Elemente (7) aufweist.

4. Form nach Anspruch 2,
**dadurch gekennzeichnet, dass**
zu jedem komplementären Element (7) der zweiten Teilform mehrere nebeneinander angeordnete Rastelemente (11) der ersten Teilform (2) vorhanden sind.

5. Form nach einem der vorhergehenden Ansprüche,
**dadurch gekennzeichnet, dass**
jede Teilform (2,3) aus mindestens zwei Elementen besteht.

6. Form nach Anspruch 7,
**dadurch gekennzeichnet, dass**
jede Teilform (2,3) aus zwei Halbschaleiementen besteht.

7. Form nach Anspruch 5 oder 6,
**dadurch gekennzeichnet, dass**
die Elemente einer Teilform (2,3) umlaufende aufeinander passende Verbindungsbereiche (8,9) aufweisen, die durch lösbare Befestigungsmittel (10) miteinander verbunden werden können.

8. Form nach Anspruch 7,
**dadurch gekennzeichnet, dass**
es sich bei den Befestigungsmitteln eines Elementes einer Teilform (2,3) um Öffnungen handelt, in die Haken (10) oder Pilzköpfe eines anderen Elementes der Teilform (2,3) greifen.

## Claims

1. Mould for producing spacers for endoprostheses containing at least two sub-moulds,
whereby
the first sub-mould (2) comprises, on an open side, a receiving section (4) into which an insertion section (5) on the open side of the second sub-mould (3) can be inserted,
whereby the first sub-mould (2) comprises connecting elements (11) that are provided to be integrated into the first sub-mould (2),
whereby the mould serves for producing a spacer for a hip endoprosthesis,
whereby one of the two sub-moulds (2, 3) surrounds the shape of a femoral head with femoral neck base and the other sub-mould (3, 2) surrounds the shape of a stem with femoral head base,
whereby a plurality of connecting elements is provided on the second sub-mould (3), and whereby the receiving section (4) is being pushed over the insertion section (5),
**characterised in that**
the connecting elements (11) of the first sub-mould (2) engage the outer circumference of the second sub-mould and can hold the second sub-mould (3) in position by means of a form-fitting and/or force-locking connection, such that the receiving section (4) can be affixed in different positions and can thus be adapted to the length of the femoral neck or such that spring elements are provided on the receiving section (4) of the first sub-mould and the second sub-mould does not comprise complementary elements.

2. Mould according to claim 1,
**characterised in that**
the connecting elements (11) are snap-in elements that fit into complementary elements (7) on the second sub-mould (3).

3. Mould according to claim 2,
**characterised in that**
the second sub-mould (3) comprises, other for each snap-in element (11) of the first sub-mould (2), multiple complementary elements (7) that are arranged adjacent to each.

4. Mould according to claim 2,
**characterised in that**
multiple snap-in elements (11) of the first sub-mould (2) that are arranged adjacent to each are present for each complementary element (7) of the second sub-mould.

5. Mould according to any one of the preceding claims,
**characterised in that**
each sub-mould (2, 3) consists of at least two elements.

6. Mould according to claim 7,
**characterised in that**
each sub-mould (2, 3) consists of two semi-shell elements.

7. Mould according to claim 5 or 6,
**characterised in that**
the elements of a sub-mould (2, 3) comprise circumferential connecting regions (8, 9) that fit onto each other and can be connected to each other by means of detachable fastening means (10).

8. Mould according to claim 7,
**characterised in that**
the fastening means of an element of a sub-mould (2, 3) are openings that are engaged by hooks (10) or mushroom-shaped heads of another element of the sub-mould (2, 3).

## Revendications

1. Moule pour la fabrication d'écarteurs pour endoprothèses contenant au moins deux moules partiels,
dans lequel le premier moule partiel (2) présente sur un côté ouvert une section de réception (4) dans laquelle une section d'insertion (5) sur le côté ouvert du second moule partiel (3) peut être insérée,
dans lequel le premier moule partiel (2) présente des éléments de connexion (11) qui sont réalisés de manière intégrée dans le premier moule partiel (2), dans lequel le moule sert à la fabrication d'un écarteur pour une endoprothèse de hanche, dans lequel un des deux moules partiels (2, 3) entoure le moule d'une tête fémorale avec une rallonge de col fémoral et l'autre moule partiel (3,2) entoure le moule d'un arbre avec une rallonge de col fémoral,
dans lequel une pluralité d'éléments de connexion est réalisée sur le second moule partiel (3), et dans lequel la section de réception (4) est poussée au-delà de la section d'insertion (5),
**caractérisé en ce que**
les éléments de connexion (11) du premier moule partiel (2) sont appliqués sur la périphérie externe du second moule partiel et peuvent maintenir le second moule partiel (3) en position par le biais d'une connexion par conjugaison de formes et/ou par adhérence de telle sorte que la section de réception (4) peut être fixée dans différentes positions et peut ainsi être adaptée à la longueur du col du fémur ou de telle sorte que des éléments élastiques sont réalisés sur la section de réception (4) du premier moule partiel et le second moule partiel ne présente pas d'éléments complémentaires.

2. Moule selon la revendication 1,
**caractérisé en ce que**
les éléments de connexion (11) sont des éléments d'encliquetage qui s'adaptent au second moule partiel (3) dans des éléments complémentaires (7).

3. Moule selon la revendication 2,
**caractérisé en ce que**
le second moule partiel (3) présente plusieurs éléments complémentaires (7) disposés les uns à côté des autres pour chaque élément d'encliquetage (11) du premier moule partiel (2).

4. Moule selon la revendication 2,
**caractérisé en ce que**
plusieurs éléments d'encliquetage (11) disposés les uns à côté des autres du premier moule partiel (2) sont présents pour chaque élément complémentaire (7) du second moule partiel.

5. Moule selon une des revendications précédentes,
**caractérisé en ce que**
chaque moule partiel (2, 3) se compose d'au moins deux éléments.

6. Moule selon la revendication 7,[JT1]
**caractérisé en ce que**
chaque moule partiel (2, 3) se compose de deux éléments de demie coque.

7. Moule selon la revendication 5 ou 6,
**caractérisé en ce que**
les éléments d'un moule partiel (2, 3) présentent des régions de connexion périphériques (8, 9) adaptées l'une à l'autre qui peuvent être reliées ensemble par des moyens de fixation amovibles (10).

8. Moule selon la revendication 7,
**caractérisé en ce que**
il s'agit pour les moyens de fixation d'un élément d'un moule partiel (2, 3) d'ouvertures dans lesquelles des crochets (10) ou têtes bombées d'un autre élément du moule partiel (2, 3) s'engagent.
